Europäisches Patentamt

European Patent Office

Office européen des brevets

(11) Veröffentlichungsnummer: **0 230 294**
**A2**

# (12) EUROPÄISCHE PATENTANMELDUNG

(21) Anmeldenummer: 87100591.4

(22) Anmeldetag: 17.01.87

(51) Int. Cl.⁴: **A 61 M 31/00**
A 61 C 5/08

(30) Priorität: 22.01.86 DE 3601787

(43) Veröffentlichungstag der Anmeldung:
29.07.87 Patentblatt 87/31

(84) Benannte Vertragsstaaten:
AT BE CH DE ES FR GB GR IT LI LU NL SE

(71) Anmelder: Cox, Kirsten, Dr.
Gartenstrasse 3
D-4057 Brüggen 2 (DE)

Wiegner, Georg
Breslauer Strasse 35
D-4060 Viersen 11 (DE)

(72) Erfinder: Cox, Kirsten
Gartenstrasse 3
D-4057 Brüggen 2 (DE)

(74) Vertreter: Uhlmann, Hans, Dr. rer.nat., Dipl.-Chem.
Gladbacher Strasse 189
D-4060 Viersen 1 (DE)

(54) Applikator zum Applizieren von Wirkstoffen.

(57) Der Applikator ist bestimmt zum Applizieren von Wirkstoffen wie Medikamenten, Antibiotika, Genußmitteln, Mundpflegemitteln u. ä. im Mundbereich und hat die Form eines in Form und Abmessung weitgehend der Außenform der Zahnkrone eines oder mehrerer natürlicher Zähne entsprechenden Hohlkörpers, der die Wirkstoffe aufnimmt. Buccal und/oder palatinal bzw. lingual sind in dem Hohlkörper Durchtrittsöffnungen zum Durchtritt des Speichels und der darin aufgelösten Wirkstoffe in den Mundraum vorhanden.

Fig. 1

## Beschreibung

Applikator zum Applizieren von Wirkstoffen

Die Erfindung betrifft einen Applikator zum Applizieren von Wirkstoffen, wie Medikamenten, Antibiotika, Genußmitteln, Mundpflegemitteln u.ä. im Mundbereich und für diese Applikation vorgesehene Gebilde.

Für eine Vielzahl von Wirkstoffen ist es erwünscht, daß diese im Körper über einen möglichst langen Zeitraum in einer bestimmten Dosierung vorliegen und verbrauchte oder abgebaute Wirkstoffe möglichst im Maße des Abbaus bzw. Verbrauchs wieder ersetzt werden, so daß ein möglichst gleichmäßiger Spiegel der Wirkstoffe im Organismus vorhanden ist. Typische Beispiele, bei denen es ganz besonders auf einen möglichst gleichmäßigen Spiegel der Wirkstoffe im Organismus ankommt, sind Antibiotika, Herzglykoside, schmerzstillende Mittel und Hormone. So mußte im Frühstadium der Anwendung von Penicillin dieses zur Aufrechterhaltung eines wirksamen Penicillinspiegels im kurzen Zeitraum von 1 bis 2 Stunden dem Patienten verabreicht werden – und das auch über Nacht.

Es sind bereits viele Vorschläge gemacht und realisiert worden, um diesem Bedürfnis nach einer möglichst gleichmäßigen Verabreichung von Wirkstoffen Rechnung zu tragen. So werden Medikamente mit einer Umhüllung versehen, die sich beispielsweise im Magen-oder Darmtrakt nur allmählich auflösen. Andere Versuche gehen dahin, durch chemische Veränderung den Abbau eines Wirkstoffes im menschlichen oder tierischen Körper zu verlangsamen und damit zu einem möglichst langanhaltenden und gleichmäßigen Wirkstoffspiegel zu führen. Weitere Möglichkeiten sind die Tropfinfusion, die subkutane Applizierung oder aber die Verabreichung in Form von Lutsch- bzw. Sublingualtabletten.

All den bisher bekannten Applikationsformen mit diesem Ziel haftet der Nachteil an, daß sie diese Aufgabe entweder nur ungenügend erfüllen, wie das bei Lutsch-Sublingualtabletten der Fall ist, oder aber, daß sie in ihrer Anwendung aufwendig und unbequem sind und häufig nur bei stationärer Behandlung durchgeführt werden können. Das gilt z.B. für die subkutane Anwendung und die Tropfinfusion.

Das gilt auch für einen in der DE-OS 32 10 242 gemachten Vorschlag, der dahin geht, in den Knochen des Kiefers ein Zahnimplantat einzubringen, in das Austrittsöffnungen eingebracht sind, durch die hindurch Medikamente direkt in die Blutbahn abgegeben werden. Dabei können die Austrittsöffnungen reguliert und so die Abgabemenge des Medikaments beeinflußt werden. Nach oben ist das Implantat und vor allem das darin für die Medikamenteneingabe mittels einer Spritze vorgesehene Ventil mit einer lösbaren Schutzeinrichtung geschützt. Daß dieser Vorschlag keinen Eingang in die Praxis gefunden hat und papierener Stand der Technik geblieben ist, ist vor allem darin begründet, daß das Setzen eines Implantates einen erheblichen operativen Eingriff im Kiefer erfordert, den ein Patient nur ungern in Kauf nimmt und der nur von einer begrenzten Anzahl von Zahnärzten beherrscht wird. Er erfordert große Geschicklichkeit, besondere Werkzeuge und ist durch seine Kompliziertheit sehr kostenaufwendig. Außerdem ist ein solches Zahnimplantat ein Fremdkörper, bei dem es Probleme mit dem Einwachsen gibt und das sich häufig nach gewisser Zeit wieder lockert. Im hier vorliegenden speziellen Fall kommt hinzu, daß der Knochen, der sich gegen Fremdkörper aktiv mit Bildung neuer Knochensubstanz wehrt, durch Knochenneubildung leicht die Austrittsöffnungen zusetzt und damit eine sichere Abgabe von Medikamenten nicht mehr gewährleistet ist. Außerdem ist der als Zahnimplantat ausgebildete Applikator in seiner Anwendung auf die wenigen Medikamente beschränkt, die unmittelbar ins Blut gelangen sollen und hierfür genügend knochengängig sein müssen. Er ermöglicht nicht die dosierte Abgabe von Wirkstoffen, die den Mund- und Rachenraum erreichen sollen, wie Mundpflegemittel oder Medikamente, die über die Schleimhäute aufgenommen werden sollen oder solche, die zur Heilung von Erkrankungen der Mund- und Rachenräume bestimmt sind.

Der Erfindung liegt die Aufgabe zugrunde, hier Abhilfe zu schaffen und zu den vorbekannten Applikationsformen der peroralen Verabreichung von Tabletten, Tropfen oder Kapseln, der rektalen Verabreichung in Form von Zäpfchen und Kapseln, der Injektion bzw. Infusion von Flüssigkeiten, der Verabreichung in Form von Aerosolen über die Lunge, der Verabreichung über die Haut in Form von Seifen, Pasten oder Lotions bzw. der Implantierung unter die Haut durch subkutane Anwendung oder der Abgabe direkt ins Blut über Zahnimplantate eine gänzlich neue Applikationsform hinzuzufügen, die bequem und sicher in der Anwendung ist und es ermöglicht, ohne großen operativen Eindruck Wirkstoffe in gleichmäßiger Dosierung über einen langen Zeitraum dem Organismus zuzuführen.

Diese Aufgabe wird gelöst durch einen Applikator zum Applizieren von Wirkstoffen, wie Medikamenten, Antibiotika, Genußmitteln, Mundpflegemitteln u.ä. im Mundbereich in Form eines hohlraumförmigen Gebildes, in das der Wirkstoff einbringbar ist und das Durchtrittsöffnungen zur gezielten Abgabe bestimmter Wirkstoffmengen aufweist, der dadurch gekennzeichnet ist, daß das hohlraumförmige Gebilde in Form und Abmessung weitgehend der Außenform der Zahnkrone eines oder mehrerer natürlicher Zähne entspricht und buccal und/oder palatinal bzw. lingual mindestens eine Durchtrittsöffnung zum Durchtritt des Speichels und der darin aufgelösten Wirkstoffe in den Mundraum aufweist. Diese Durchtrittsöffnungen können ganz unterschiedlich gestaltet sein. In ihrer einfachsten und auch in optimaler Form sind es einfach runde Löcher. Sie können zweckmäßig aber auch als Schlitze ausgeführt sein, insbesondere dann, wenn diese vorteilhaft durch einen Schieber verschließbar und dadurch auf verschiedene Öffnungsgrößen einstellbar sind.

Der besondere Vorteil des erfindungsgemäßen Applikators liegt darin, daß damit die Abgabe von Wirkstoffen in den Mund- und Rachenraum möglich ist und daß mit Hilfe der Anzahl, größe oder Form der Zutrittsöffnungen die Menge des Speichels dosiert werden kann, die für die Herauslösung und Weitergabe der Wirkstoffe in den Mund- und Rachenraum zur Verfügung steht. Ein weiterer besonderer Vorteil liegt darin, daß das hohlraumförmige Gebilde selbst im Mundbereich auf sehr einfache Art und Weise untergebracht werden kann und daß der Patient beispielsweise nur einmal am Tag, z.B. früh nach dem Zähneputzen, oder einmal pro Woche den Wirkstoff in dieses hohlraumförmige Gebilde in geeigneter Form einbringt und dieser dann nach und nach an den Organismus abgegeben wird. Dabei ist der Mundraum als Applikationsort ohnehin bevorzugt, weil bekanntlich über die Schleimhäute Wirkstoffe bevorzugt aufgenommen werden bzw. dort zur Verfügung stehen sollen, wie das bei Mundpflegemitteln bzw. Medikamenten für den Hals-und Rachenraum der Fall ist. Ein weiterer Vorteil liegt darin, daß, bis auf die einmalige Einbringung des hohlraumförmigen Gebildes in einer später noch zu beschreibenden Art und Weise, keinerlei Eingriffe notwendig sind und sich das hohlraumförmige Gebilde an Stellen unterbringen läßt, die ohnedies bei den meisten Menschen ungenutzt zur Verfügung stehen.

Bevorzugt ist eine Ausführungsform der Erfindung, bei der das hohlraumförmige Gebilde sich in einer vorhandenen Zahnlücke befindet, wie sie viele Menschen leider bereits seit ihrer frühesten Jugend haben. Besonders vorteilhaft ist ein hohlraumförmiges Gebilde, das in einer Ausbohrung eines defekten Zahnes untergebracht werden kann, so daß sich der unangenehme Besuch beim Zahnarzt mit der positiven Nebenwirkung verbinden läßt, für die Zeit des übrigen Lebens eine Depotmöglichkeit für Wirkstoffe geschaffen zu haben. Dabei können die hohlraumförmigen Gebilde bei einem Prothesenträger bevorzugt Bestandteil einer partiellen oder totalen Zahnprothese oder einer Brücke sein.

Das hohlraumförmige Gebilde als Bestandteil einer Brücke ermöglicht einen besonders einfach zu gestaltenden und zu handhabenden Applikator, indem die immer erforderliche Öffnung bzw. Öffnungsmöglichkeit zum Einbringen des Wirkstoffs als Loch oder Schlitz an mindestens einer Seitenwand angebracht ist und der Verschluß der Öffnung auf einfache Weise beim Verankern der Brücke dadurch bewirkt wird, daß die Öffnung durch einen der beiden Verankerungselemente verschlossen wird. Der Vorteil dieser Ausführungsform liegt darin, daß keinerlei zusätzliche mechanische Mittel wie Scharniere zum Öffnen und Verschließen erforderlich sind.

Eine weitere vorteilhafte Ausgestaltung der Erfindung besteht in einem hohlraumförmigen Gebilde als Aufsatzteil, das auf dem Stumpf eines Zahnes ausgebildet ist, der bis nahe an die Pulpa abgearbeitet ist. Auf diese Weise läßt sich auch der Personenkreis für das neue Applizierungsverfahren erschließen, der keine Zahnlücke oder eine Teil- bzw. Totalprothese hat.

Bei einem gesunden, eigens zur Verwirklichung der Erfindung bis nahe an die Pulpa mit einem Normalwerkzeug abgearbeiteten Zahn ist der Applikator bevorzugt als Doppelkrone ausgebildet, bei der zweckmäßig die Doppelkrone aus Innenkrone und Außenkrone besteht, wobei die Innenkrone auf dem Zahnstumpf befestigt ist und die Außenkrone auf der Innenkrone aufsitzt. Innenkrone und Außenkrone umschließen die Wirkstoffkammer, in der das Wirkstoffpräparat untergebracht ist. Der Zugang zur Wirkstoffkammer läßt sich auf unterschiedliche Art und Weise realisieren. Eine einfache und bevorzugte Lösung besteht darin, daß die Außenkrone zum Einbringen des Wirkstoffpräparates einfach nach oben abgezogen, das Wirkstoffpräparat auf die Innenkrone aufgebracht und dann die Außenkrone wieder aufgeschoben wird. Bei einem solchen Applikator ist der Zahn bevorzugt so abgearbeitet, daß die Oberfläche des Zahnstumpfes konkav ausgebildet ist und das Wirkstoffpräparat dadurch in der ebenfalls konkav gehaltenen Innenkrone festgehalten wird und nicht wegrollen kann.

Eine andere Möglichkeit des Zugangs zur Wirkstoffkammer und des Einbringens des Wirkstoffpräparates besteht darin, daß die Außenkrone aus einem Außenmantel und einem Kaufflächenteil besteht, wobei letzterer der Kaufläche des ursprünglichen Zahnes entspricht und das Kaufflächenteil über ein Scharnier mit dem Außenmantel aufklappbar verbunden ist.

Eine weitere vorteilhafte Ausführungsform des Applikators und der Möglichkeit des Einbringens des Wirkstoffpräparates besteht darin, daß der Applikator als Geschiebe ausgebildet ist, so daß das an einer Seite offene, sonst aber allseits geschlossene hohlraumförmige Gebilde zweckmäßig nach oben bzw. unten oder aber auch nach innen oder außen bis zu einem Anschlag geschoben werden kann und in dieser geöffneten Stellung das Wirkstoffpräparat eingeführt wird.

Bei einem devitalen Zahn hat sich eine abgewandelte Applikatorform als vorteilhaft erwiesen. Die Innenkrone ist in diesem Fall als nach oben offener Topf ausgebildet, der mit Hilfe von in die Wurzelkanäle eingreifenden Schrauben in den Wurzeln des Zahnes befestigt ist. Auf diese topfartige Innenkrone ist wiederum die Außenkrone aufgesetzt. In diesem Fall weisen sowohl Innen- als auch Außenkrone Durchtrittsöffnungen auf, wobei die der Innenkrone größer sind als diejenigen der Außenkrone. Durch Verdrehen beider ist die Größe der Durchtrittsöffnung insgesamt einstellbar und damit eine Dosierung des Wirkstoffpräparates möglich.

In einer vorteilhaften weiteren Ausführungsform der Erfindung hat der Applikator die Form eines hohlen Kunststoffzahnes, der zweiteilig ausgebildet ist und aus Kaufflächenteil und Basisteil besteht, wobei das Basisteil das Wirkstoffpräparat aufnimmt und die Öffnungen zum Durchtritt des Speichels aufweist.

Zusätzlich zu der Steuerung der für den Organismus freigesetzten Menge an Wirkstoffen über Größe, Form und Anzahl der Durchtrittsöffnungen können zweckmäßig zusätzliche Maßnahmen zur Verlängerung der Wirkungsdauer ergriffen werden. Eine vorteilhafte Maßnahme besteht darin, daß die in das hohlraumförmige Gebilde eingebrachten Wirkstoffe durch chemische Umsetzung gegenüber dem als Transportmedium wirkenden Speichel gezielt widerstandsfähig gemacht sind. Eine weitere bevorzugte Möglichkeit besteht darin, daß dem in dem hohlraumförmigen Gebilde befindlichen Wirkstoff Inertstoffe zugemischt sind, durch die der Zugriff des Speichels verlangsamt wird. Auch hat es sich als zweckmäßig erwiesen, die in dem hohlraumförmigen Gebilde befindlichen Wirkstoffe mit einer Umhüllung zu versehen, durch die sie gegenüber dem Angriff des Speichels gezielt widerstandsfähig gemacht sind.

Die Erfindung wird nachfolgend anhand der Zeichnungen in einigen typischen und vorteilhaften Ausführungsformen näher erläutert, ohne daß sie jedoch auf die konkret dargestellten Ausführungsbeispiele beschränkt ist.

Figuren 1 bis 6 zeigen zwei verschiedene Ausführungen von Applikatoren in Form einer Doppelkrone in Verbindung mit einem vitalen Zahn bei gesunder Pulpa,

die Figuren 7 bis 9 einen Applikator in Verbindung mit einem devitalen Zahn,

die Figuren 10 bis 13 einen Applikator untergebracht in einer Zahnlücke als Bestandteil einer Brücke,

die Figur 14 einen Doppelapplikator als Bestandteil einer Teilprothese,

die Figuren 15 und 16 einen Applikator in Verbindung mit einem Zahnimplantat,

die Figuren 17 bis 19 einen Applikator als Doppelkrone mit Hebelbefestigungen,

Figur 20 bis 24 einen Kunststoffzahn ausgebildet als Applikator.

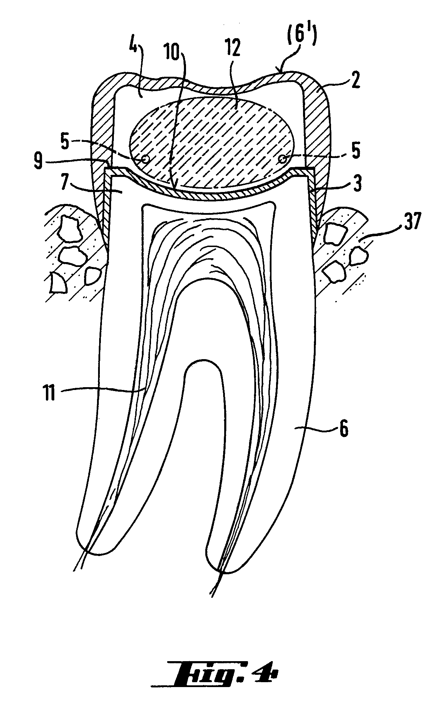

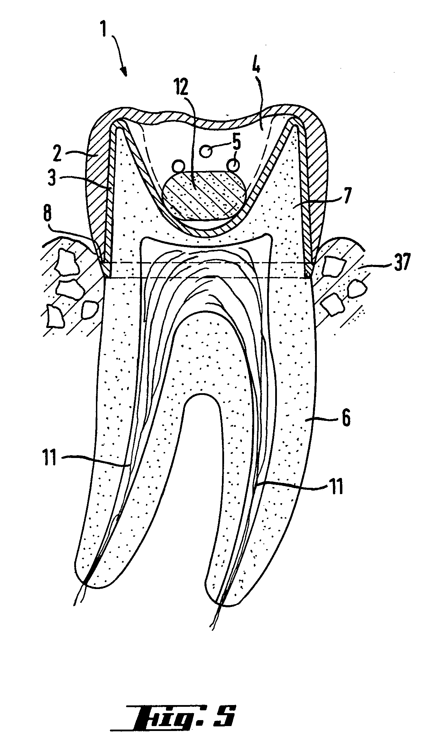

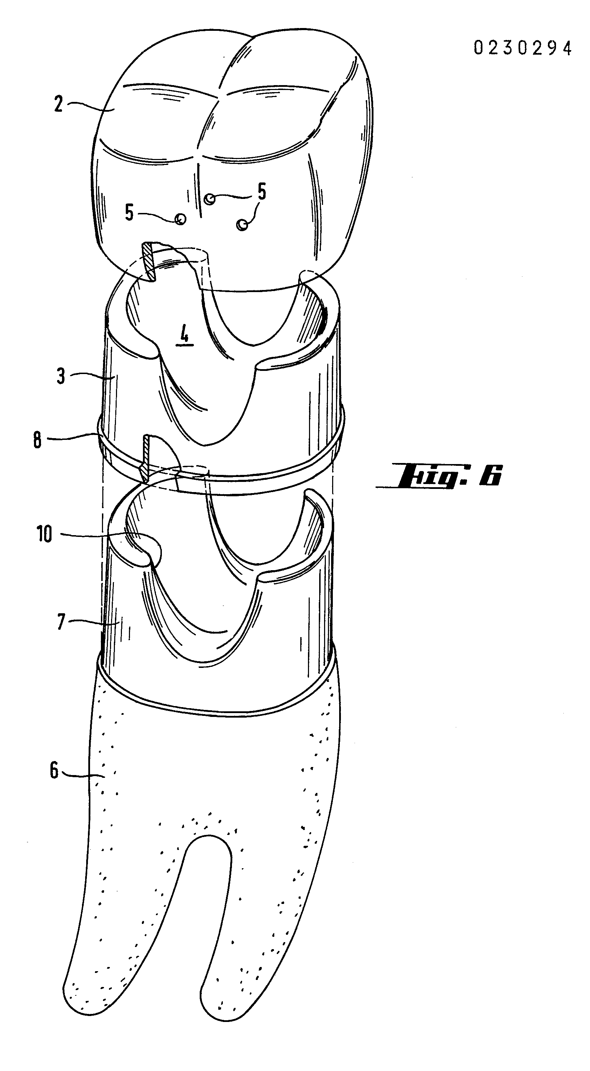

Die Figuren 4 bis 6 zeigen die einfachste Ausführungsform eines Applikators in Form einer Doppelkrone 1, und zwar Figur 4 und 5 Seitenansichten im Schnitt, Figur 6 das Explosionsbild zu Figur 5. Der vitale Zahn 6 ist bis dicht oberhalb der Pulpa 11 mit einem eigens für diese Applikatoren entwickelten Normbohrer abgearbeitet. Auf dem Zahnstumpf 7, dessen obere Auschlußfläche 10 konkav ausgebildet ist, ist die Innenkrone 3 aufzementiert. Auf die Innenkrone 3 ist die innen hohle und mit Durchtrittsöffnungen 5 für den Speichel und die herausgelösten Wirkstoffe versehene Außenkrone 2 aufgesetzt. Innenkrone 3 und Außenkrone 2, die in ihrem oberen Bereich der ursprünglichen Kaufläche des natürlichen Zahnes 6' entsprechen, schließen die zur Aufnahme des Wirkstoffpräparates 12 bestimmte Wirkstoffkammer 4 ein und bilden zusammen den Applikator.

Die Außenkrone 2 weist in Figur 4 an ihrer inneren seitlichen Umfangsfläche eine Stufe 9 auf, so daß die Außenkrone 2 nicht nur von der Seite her auf der Innenkrone 3 gehalten wird, sondern auch von oben her in ihrer Lage auf der Innenkrone 3 fixiert ist. Außerdem ist es dadurch möglich, die Wandstärke der Außenkrone 2 stärker zu halten, ohne daß das zu einer Störung der anatomischen Gegebenheiten im Mundraum führt.

In der Ausführungsform nach Figur 5 und 6 befindet sich der Anschlag für die Außenkrone 2 als Stufe 8 an der Innenkrone 3. Diese Ausführungsform hat den Vorteil, daß die Außenkrone 2 in verhältnismäßig dünner Wandstärke ausgeführt werden und in ihrem Gewicht leicht gehalten werden kann. Bei diesem Ausführungsbeispiel ist der Zahnstumpf 7 so ausgearbeitet, daß das Zahnbein im Bereich zu benachbarten Zähnen verhältnismäßig hoch erhalten worden ist, so daß die darauf aufgebrachte Innenkrone 3 in diesem Bereich als Stütze für die Außenkrone 2 dient. Nach innen und außen, d.h. buccal und palatinal bzw lingual ist der Zahnstumpf 7 dagegen weiter abgearbeitet. Durch diese unterschiedliche Höhe des Außenumfanges des Zahnstumpfes 7 wird einerseits eine sichere Friktion der Außenkrone 7 und ebenso eine sichere Lagerung des Wirkstoffpräparates 12 in der Wirkstoffkammer 4 bewirkt, andererseits haben nach innen und außen, d.h. buccal und palatinal bzw. lingual die Durchtrittsöffnungen 5 freie Verbindung zur Wirkstoffkammer 4, so daß der Durchtritt des Speichels und die Abgabe der Wirkstoffe mit Hilfe des Speichels gewährleistet ist.

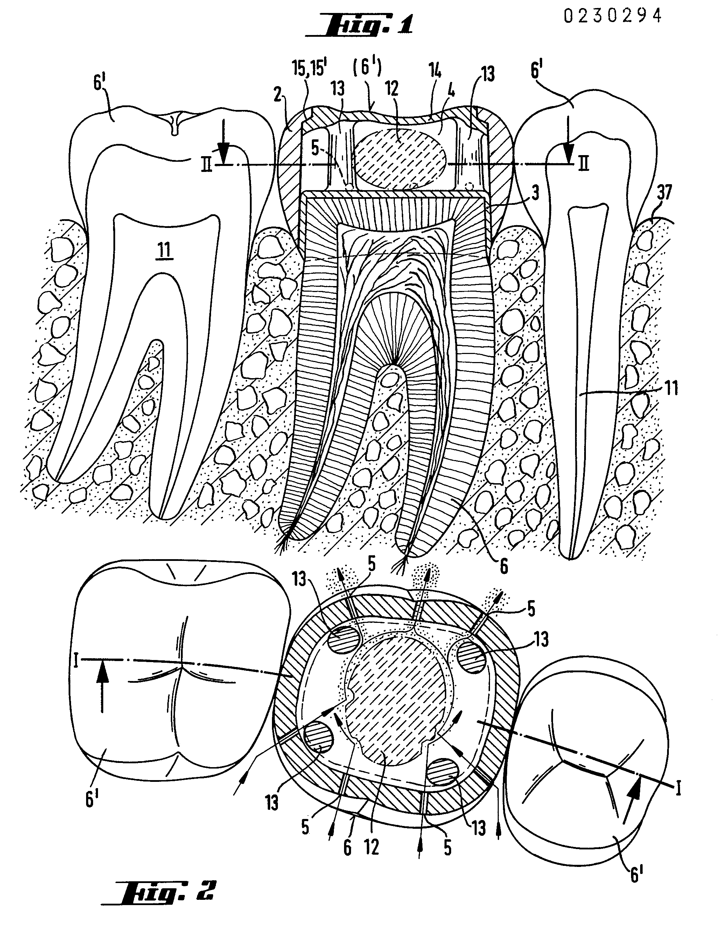

Der Applikator gemäß Figur 1 bis 3 unterscheidet sich von dem der Figuren 4 bis 6 dadurch, daß die Innenkrone 3 Hockerform aufweist und mit Stützpfeilern 13 mit dem Kauflächenteil 14 zu einem Stück verbunden ist, wobei die Oberfläche des Kauflächenteils 14 der Kaufläche des ursprünglich natürlichen Zahnes 6' entspricht. Die in diesem Fall offene Außenkrone 2 ist mantelförmig ausgebildet und lediglich oben bei 15' etwas eingezogen, so daß sie nach Einfügung des Wirkstoffpräparates 12 z.B. in Form einer Pille zwischen die Stützpfeiler 13 auf das Kauflächenteil 14 und die Innenkrone 3 aufgesetzt wird, wobei der eingezogene Teil 15' der offenen Außenkrone 2 zur Auflage auf der Stufe 15 des Kauflächenteils 14 kommt. Figur 1 zeigt dabei im Längsschnitt einen entsprechend abgearbeiteten natürlichen Zahn 6 zwischen zwei unbearbeitet gebliebenen Zähnen 6' und die Einbettung der Zähne 6, 6' im Kieferknochen 37.

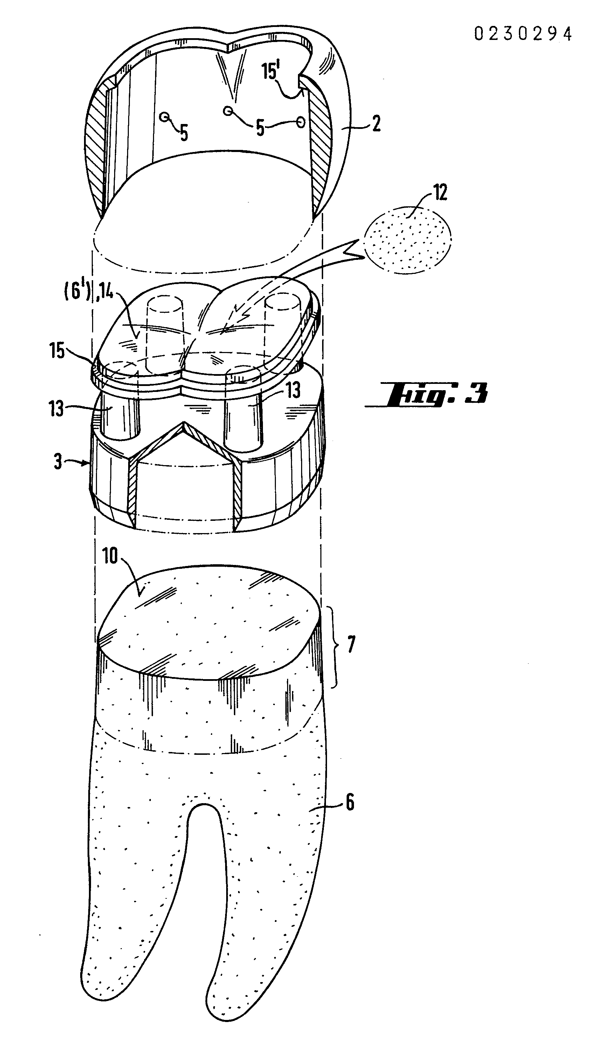

Figur 2 zeigt dieselben Zähne 6, 6' in Draufsicht im Schnitt, wobei durch die Pfeile der Angriff des Speichels durch die Durchtrittsöffnungen 5 an das Wirkstoffpräparat 12, dessen Auflösung und die Abgabe wiederum durch Pfeile durch die Durchtrittsöffnungen 5 hindurch angedeutet ist. Figur 3 ist die Darstellung desselben Applikators in perspektivischer Explosion.

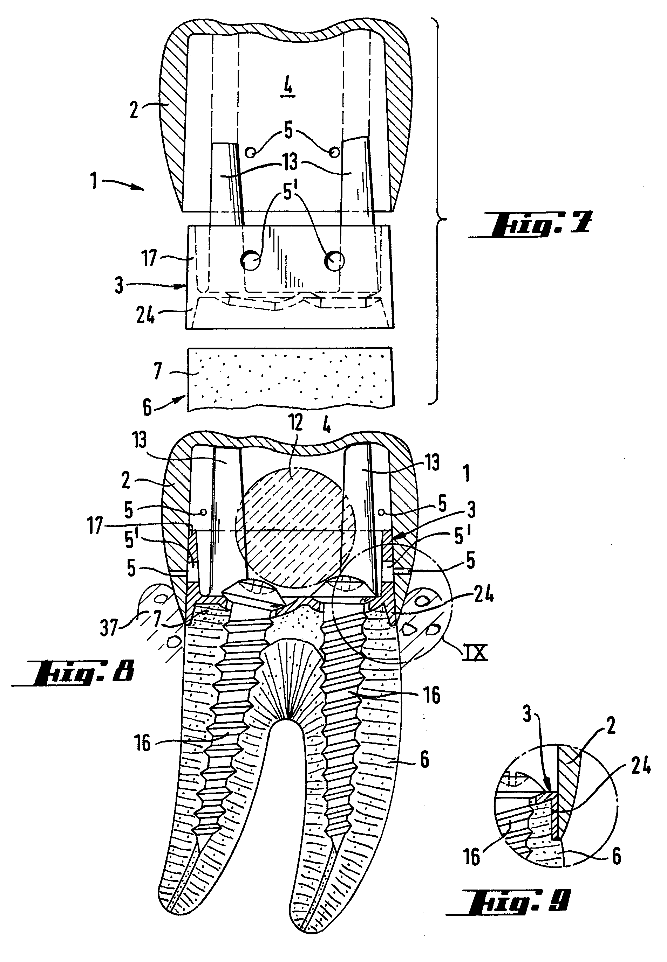

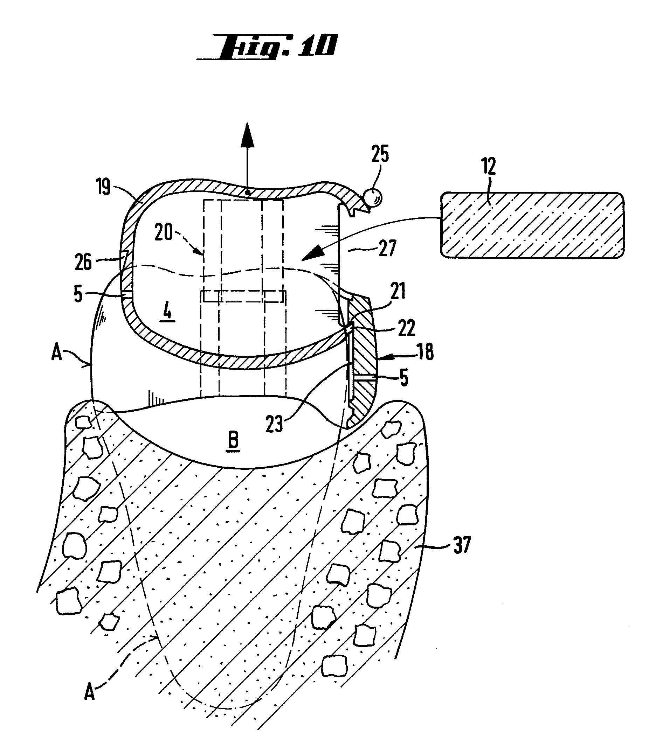

Die Figuren 7 bis 9 zeigen einen Applikator ebenfalls wieder in Form einer Doppelkrone 1 bei einem devitalen Zahn 6, bei dem der Zahnnerv entfernt ist. Der Zahn 6 ist in diesem Fall bis zum Boden des Pulpencavum abgearbeitet. Die Innenkrone 3 ist in diesem Fall als nach oben offener Topf 17 ausgebildet, der mit Hilfe von in die Wurzelkanäle eingreifenden Schrauben 16 in den Wurzeln des Zahnes 6 befestigt ist. Nach unten ist die Seitenwand der Innenkrone 3 etwas verlängert und bildet einen Kranz 24, der den Zahnstumpf 7 schützt. Auch hierbei dient die Innenkrone 3 als Halterung für die Außenkrone 2. Bei diesem Beispiel befinden sich zusätzlich zu den Durchtrittsöffnungen 5 in der Außenkrone 2 noch größer gehaltene Durchtrittsöffnungen 5' im Topf 17. Der Topf 17 weist außerdem Stützpfeiler 13 auf, auf denen die Außenkrone 2 aufsitzt, so daß die Stützpfeiler 13 als Anschlag für die Außenkrone 2 dienen. Figur 7 ist die teilweise Explosionsdarstellung zu Figur 8. Figur 9 zeigt eine Variante zur Ausführungsform der Figuren 8 und 9, bei der der Topf 17 weggelassen ist und lediglich der Kranz 24 als Sitz für die Außenkrone 2 dient, so daß sich eine ähnliche Ausbildung ergibt wie in den Figuren 1 bis 6.

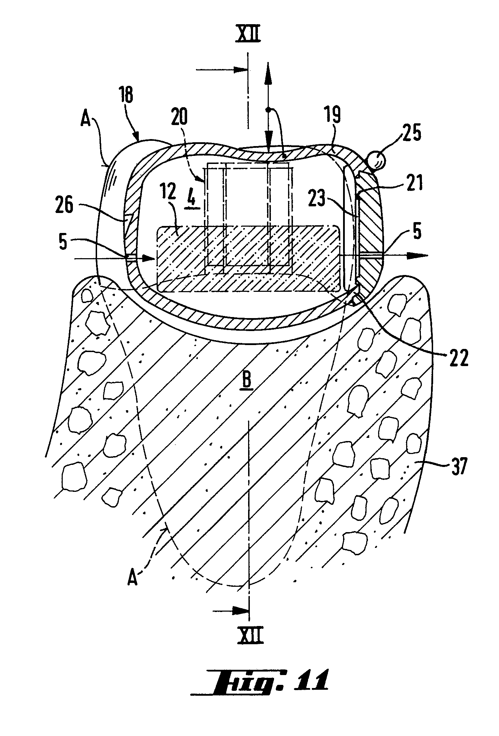

Die Figuren 10 bis 13 zeigen einen Applikator, der eine vorhandene Zahnlücke nutzt und Bestandteil einer Brücke 18 ist, die auf den Zähnen A und C verankert ist und die Zahnlücke des früheren Zahnes B überbrückt. Bestandteil dieser Brücke 18 ist das als Applikator für Wirkstoffe dienende Brückenglied 19, das seitlich zu den benachbarten Zähnen A und C ein Geschiebe 20 aufweist, so daß das Brückenglied 19 durch einen Kugelknopf 25 oder eine Kerbe 26 als Handhabe nach oben bis zu dem Anschlag 21 herausgezogen werden kann und die Öffnung 27 freigibt, durch die das Wirkstoffpräparat 12 eingeführt werden kann. Zu diesem Zweck weist das Brückenglied 19 an einer Seite die Feder 22 auf, die in der Nut 23 gleitet.

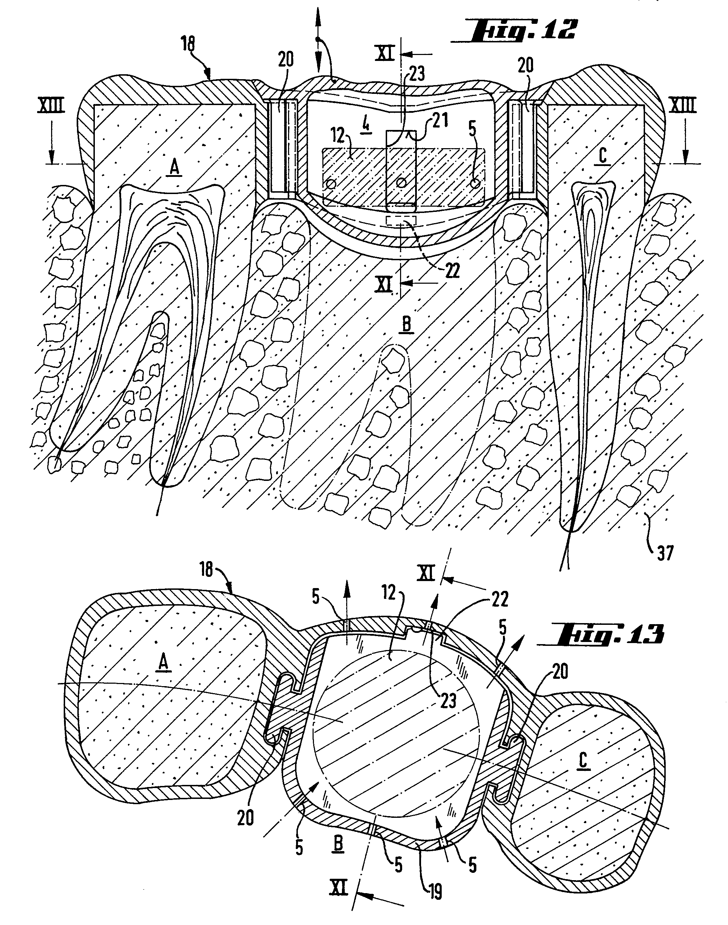

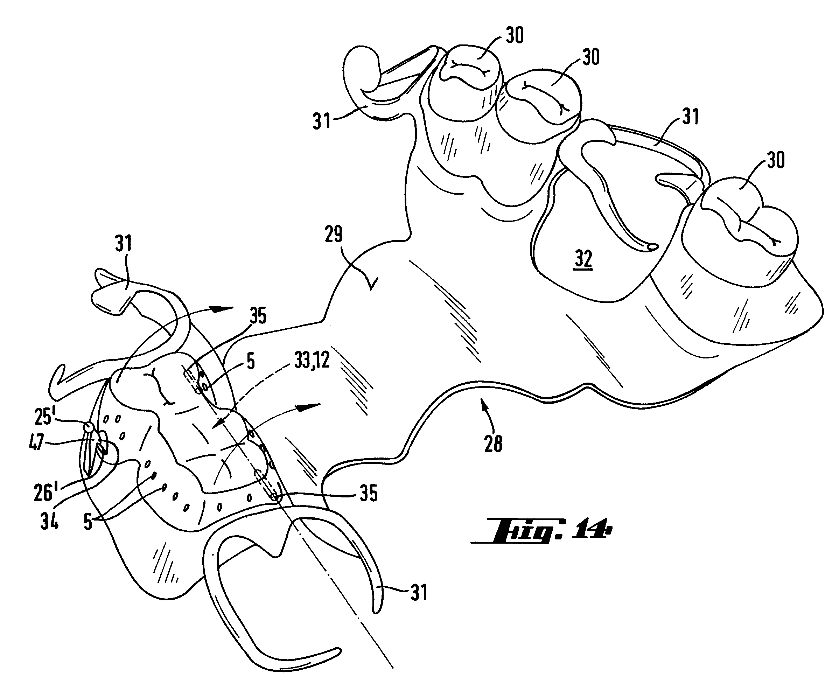

Die Figur 10 zeigt das Brückenglied 19 im geöffneten Zustand in Seitenansicht im Schnitt, Figur 11 im geschlossenen Zustand, Figur 12 die Einbeziehung des Brückengliedes 19 in die Brücke 18 und die Verankerung auf den Zähnen A und C in Seitenansicht im Schnitt, Figur 13 die Draufsicht ebenfalls im Schnitt.

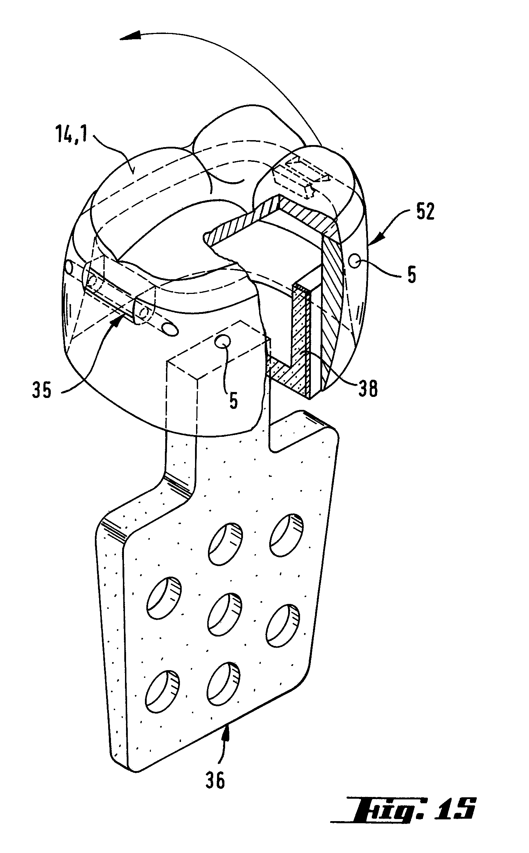

Figur 14 zeigt einen Applikator als Bestandteil einer Teilprothese 28, die aus einer Gaumenplatte 29 bzw. einem Bügel besteht, an der beidseitig künstliche Zähne 30 befestigt sind. Die Befestigung der Teilprothese 28 an nicht dargestellten, noch vorhandenen Zähnen erfolgt mittels Klammern 31. Der rechte Teil der Teilprothese 28 hat eine Aussparung 32, um Platz für den Durchtritt eines noch vorhandenen natürlichen Zahnes zu lassen. Die beiden künstlichen Zähne 30 des linken Teils der Teilprothese 28 sind als Doppelkammer 33 ausgebildet, so daß entweder größere Mengen oder auch unterschiedliche Wirkstoffpräparate 12 darin untergebracht werden können. Bei dieser Ausführungsform ist der Applikator zum Einbringen der Wirkstoffpräparate 12 über das Scharnier 35 aufklappbar, wozu ein Kugelkopf 25′ als Handhabe eines Rasthebels 47 dient und der Verschluß mittels der in die Kerbe 26′ einrastenden Raste 34 bewirkt wird.

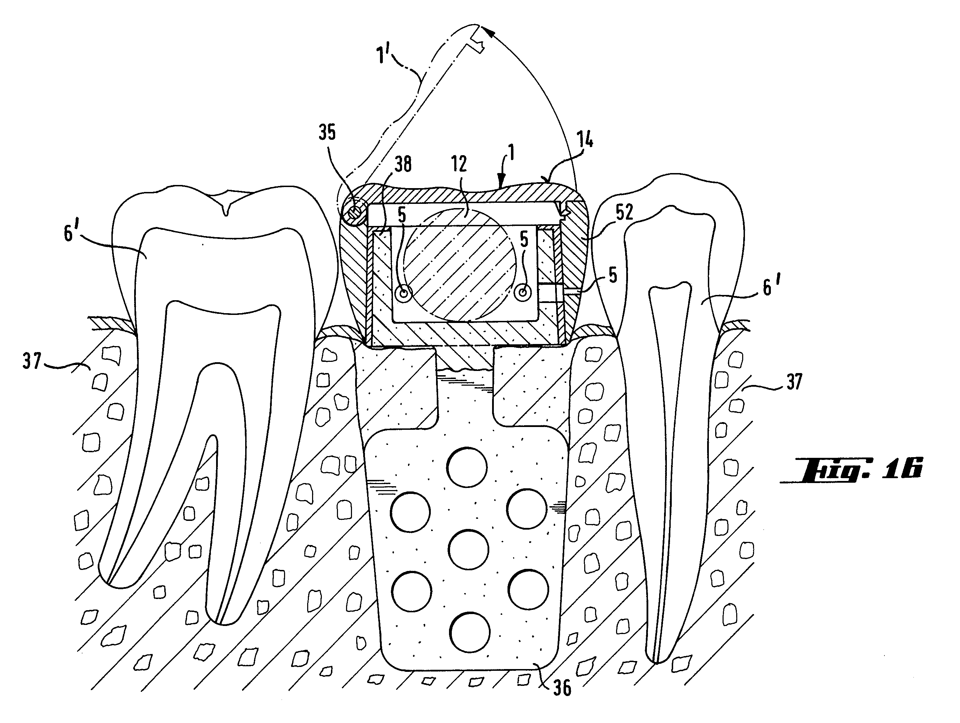

Die Figuren 15 und 16 zeigen einen Applikator in Verbindung mit einem Zahnimplantat 36 in Blattform, das in seinem aus dem Kieferknochen 37 herausragenden oberen Teil als Käfig 38 ausgebildet ist, auf dem eine hohle Krone 52 aufsitzt, wobei das Kauflächenteil 14 der Krone 52 durch ein Scharnier 35 aufgeklappt werden kann. Die Figur 15 zeigt das Zahnimplantat 36 als solches in perspektivischer Darstellung im Teilschnitt, Figur 16 in Seitenansicht im Schnitt dessen Einbettung zwischen zwei vorhandenen Zähnen 6′.

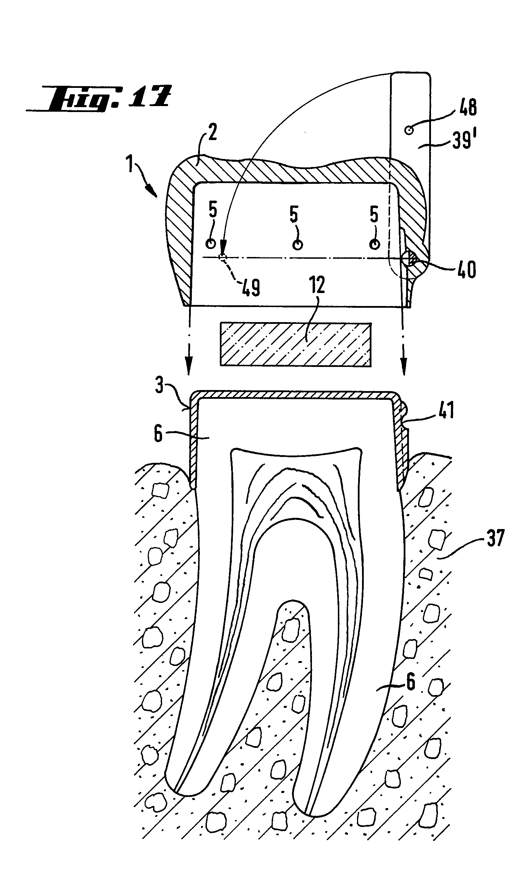

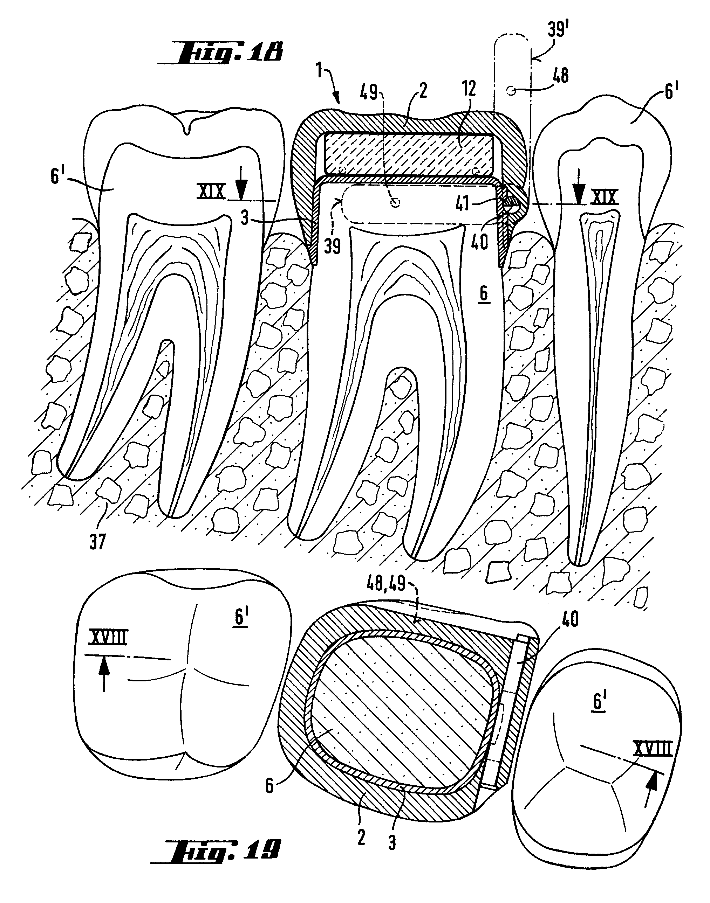

Die Figuren 17 bis 19 zeigen einen Applikator ähnlich Figur 4 bis 6 als Doppelkrone 1 mit Außenkrone 2 und Innenkrone 3 auf einem abgearbeiteten Zahn 6. Bei dieser Ausführungsform ist die Außenkrone 2 über den außen an einer Seite angreifenden Hebel 39 mit der Innenkrone 3 verbunden. Bei hochgestelltem Hebel 39, in dieser Stellung mit 39′ bezeichnet, kann die Doppelkrone 1 nach oben abgezogen werden. Nach Aufbringen des Wirkstoffpräparates 12 auf die Innenkrone 3 wird die Außenkrone 2 wieder aufgesetzt, der Klemmhebel 39 herabgedrückt und so die Außenkrone 2 auf der Innenkrone 3 arretiert. Das Klemmscharnier 40 greift dabei in die Kerbnut 41 ein und verhindert ein ungewolltes Lösen der Außenkrone 2. Am Hebel 39 ist eine Raste 48 und an der Außenkrone 2 eine Rastvertiefung 49 angebracht, welche unbeabsichtigtes Lösen des Herbels 39 verhindert. Figur 17 zeigt diese Ausführungsform in Explosionsdarstellung, Figur 18 in Seitenansicht im Schnitt im geschlossenen Zustand zusammen mit benachbarten Zähnen 6′ und Figur 19 die Draufsicht von oben teilweise geschnitten.

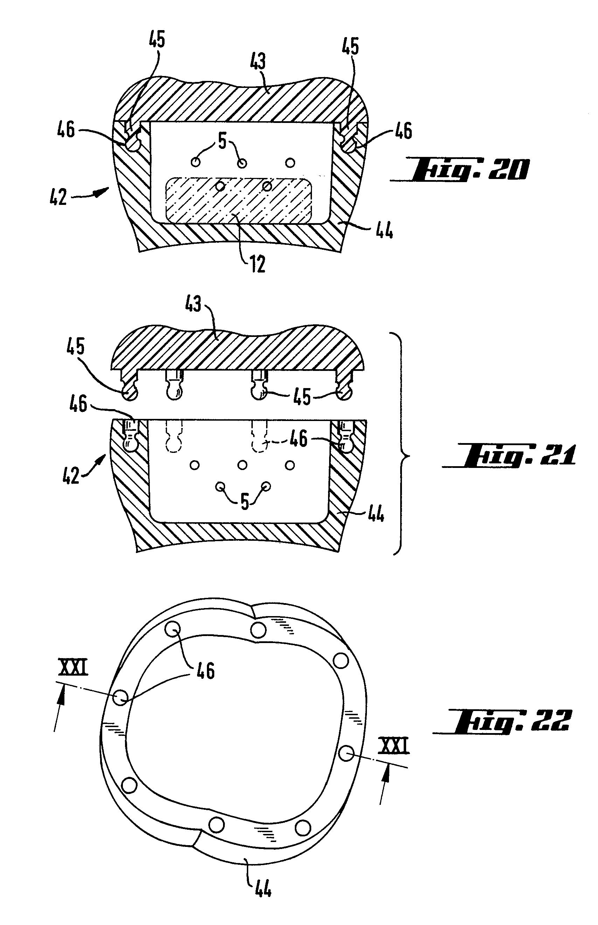

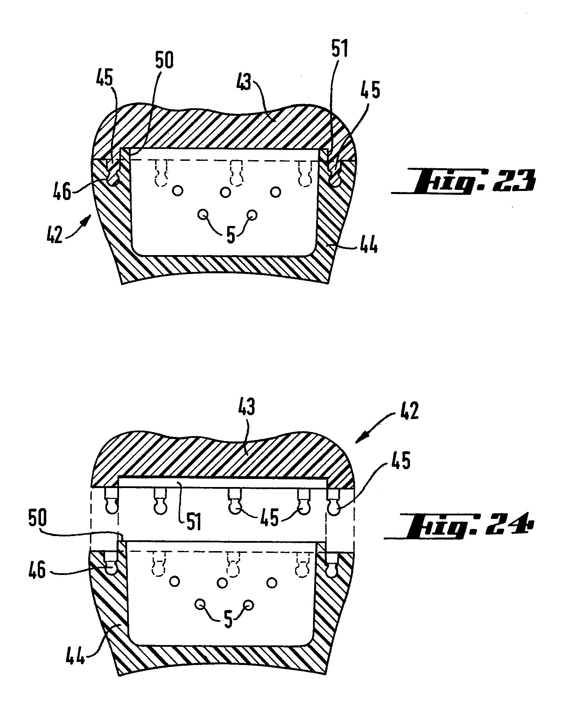

Die Figuren 20 bis 24 zeigen einen Applikator in Form eines hohlen Kunststoffzahnes 42 aus Kunststoff-Kauflächenteil 43 und Basisteil 44 zur Aufnahme des Wirkstoffpräparates 12. Kunststoff-Kauflächenteil 43 und Basisteil 44 sind über Zapfen 45, die in Napföffnungen 46 eingreifen, miteinander verbunden. Im Ausführungsbeispiel der Figuren 23 und 24 weist das Basisteil 44 einen zusätzlichen Bund 50 auf, welcher in eine entsprechende zylindrische Aussparung 51 im Kunststoff-Kauflächenteil 43 eingreift, so daß das Kunststoff-Kauflächenteil 43 zusätzlich in seiner seitlichen Lage gesichert ist.

Die Figuren 20 und 23 stellen dabei Seitenansichten im Schnitt dar, die Figuren 21 und 24 entsprechende Explosionsbilder und Figur 22 die Draufsicht von oben auf das Basisteil 44.

Als Materialien für den Applikator kommen alle aus der Zahntechnik bekannten und bewährten Materialien in Betracht wie Gold, Edelmetall reduziert, Nicht-Edelmetalle und physiologisch unbedenkliche Kunststoffe.

## Patentansprüche

1. Applikator zum Applizieren von Wirkstoffen wie Medikamenten, Antibiotika, Genußmitteln, Mundpflegemitteln u.ä. im Mundbereich in Form eines hohlraumförmigen Gebildes, in das der Wirkstoff einbringbar ist und das Durchtrittsöffnungen zur gezielten Abgabe bestimmter Wirkstoffmengen aufweist, dadurch gekennzeichnet, daß das hohlraumförmige Gebilde in Form und Abmessung weitgehend der Außenform der Zahnkrone eines oder mehrerer natürlicher Zähne entspricht und buccal und/oder palatinal bzw. lingual mindestens eine Durchtrittsöffnung (5) zum Durchtritt des Speichels und der darin aufgelösten Wirkstoffe (12) in den Mundraum aufweist.

2. Hohlraumförmiges Gebilde nach Anspruch 1, dadurch gekennzeichnet, daß das hohlraumförmige Gebilde als Einsatzteil in die Ausbohrung eines Zahnes (6) bzw. als Aufsatzteil auf den fast bis zur Pulpa abgearbeiteten Zahnstumpf (7) ausgebildet ist.

3. Hohlraumförmiges Gebilde nach einem der Ansprüche 1 und 2, dadurch gekennzeichnet, daß das hohlraumförmige Gebilde als auf den Zahnstumpf (7) aufsetzbare Doppelkrone (1) ausgebildet ist.

4. Hohlraumförmiges Gebilde nach einem der Ansprüche 1 und 2, dadurch gekennzeichnet, daß das hohlraumförmige Gebilde Bestandteil einer Brücke (18) oder Zahnprothese (28) ist.

5. Hohlraumförmiges Gebilde nach einem der Ansprüche 1 bis 4, dadurch gekennzeichnet, daß das hohlraumförmige Gebilde als Bestandteil einer Brücke an mindestens einer Seitenwand eine Öffnung zum Einbringen des Wirkstoffs aufweist, die bei Verankerung der Brücke durch die Anlage der Seitenwand am benachbarten Zahn verschlossen ist.

6. Hohlraumförmiges Gebilde nach einem der Ansprüche 1 bis 5, dadurch gekennzeichnet, daß das hohlraumförmige Gebilde durch ein zu öffnendes Kauflächenteil (14) abgeschlossen ist.

7. Hohlraumförmiges Gebilde nach einem der Ansprüche 1 bis 6, dadurch gekennzeichnet, daß das zu öffnende Kauflächenteil (14) über ein Scharnier (35) mit dem hohlraumförmigen Gebilde verbunden ist.

8. Hohlraumförmiges Gebilde nach einem der Ansprüche 1 bis 7, dadurch gekennzeichnet, daß die Doppelkrone (1) aus einer mit dem bearbeiteten Zahnstumpf (7) in Eingriff stehenden Innenkrone (3) und einer darauf aufgesetzten Außenkrone (2) besteht.

9. Hohlraumförmiges Gebilde nach Anspruch 8, dadurch gekennzeichnet, daß die Innenkrone (3) als nach oben offener Topf (17) ausgebildet ist, Durchtrittsöffnungen (5′) aufweist und die Außenkrone (2) drehbar auf der Innenkrone (3) ausgebildet ist, so daß je durch Veränderung der Lage der Außenkrone (2) zur Innenkrone (3) unterschiedliche Bereiche der Durchtrittsöffnungen (5,5′) abdeckbar sind.

10. Hohlraumförmiges Gebilde nach einem der Ansprüche 1 und 2, dadurch gekennzeichnet, daß das hohlraumförmige Gebilde als Geschiebe (20) ausgebildet ist und in geöffneter Stellung eine Öffnung (27) zur Einführung des Wirkstoffpräparates (12) frei zugängig aufweist.

11. Hohlraumförmiges Gebilde nach einem der Ansprüche 1 bis 3 und Anspruch 8, dadurch gekennzeichnet, daß die Außenkrone (2) über einen Hebel (39) lösbar auf der Innenkrone (3) befestigt ist.

12. Hohlraumförmiges Gebilde nach einem der Ansprüche 1 und 2, dadurch gekennzeichnet, daß das hohlraumförmige Gebilde ein Kunststoffzahn (42) ist, der aus Kauflächenteil (43) und Basisteil (44) zur Aufnahme des Wirkstoffpräparates (12) besteht.

13. Hohlraumförmiges Gebilde nach einem der Ansprüche 1 bis 11, dadurch gekennzeichnet, daß das hohlraumförmige Gebilde der obere Bestandteil eines Zahnimplantates (36) ist.

14. Hohlraumförmiges Gebilde nach einem der Ansprüche 1 bis 11, dadurch gekennzeichnet, daß das hohlraumförmige Gebilde auf einem Zahnimplantat (36) befestigt ist.

15. Hohlraumförmiges Gebilde nach einem der Ansprüche 1 bis 14, dadurch gekennzeichnet, daß im hohlraumförmigen Gebilde untergebrachte Wirkstoffe (12) mit einer gegenüber dem Speichel gezielt widerstandsfähig gemachten Umhüllung versehen sind.

16. Hohlraumförmiges Gebilde nach einem der Ansprüche 1 bis 15, dadurch gekennzeichnet, daß im hohlraumförmigen Gebilde untergebrachte Wirkstoffe (12) durch chemische Umsetzung gegenüber dem Speichel gezielt widerstandsfähig gemacht sind.

17. Hohlraumförmiges Gebilde nach einem der Ansprüche 1 bis 16, dadurch gekennzeichnet, daß den im hohlraumförmigen Gebilde untergebrachten Wirkstoffen (12) Inertstoffe zugemischt sind, um die Wirkstoffe gegenüber dem Speichel gezielt widerstandsfähig zu machen.

*Fig. 1*

0230294

*Fig. 2*

0230294

Fig: 3

Fig. 4

0230294

Fig. 5

0230294

2

5 5

5

4

3

8

10

7

6

*Fig: 6*

0230294

*Fig. 7*

*Fig. 8*

*Fig. 9*

0230294

Fig. 10

_Fig. 11_

0230294

**Fig. 12**

**Fig. 13**

Fig. 14

14,1

52

5

35

5

38

36

*Fig. 15*

Fig. 16

Fig. 17

0230294

**Fig. 18**

**Fig. 19**

0230294

Fig. 20

Fig. 21

Fig. 22

0230294

Fig. 23

Fig. 24